(19) Europäisches Patentamt

European Patent Office

Office européen des brevets

(11) **EP 1 400 626 A1**

(12) **EUROPEAN PATENT APPLICATION**

(43) Date of publication:
24.03.2004 Bulletin 2004/13

(51) Int Cl.⁷: **D21C 9/00**

(21) Application number: **03255804.1**

(22) Date of filing: **17.09.2003**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **19.09.2002 US 247782**

(71) Applicant: **Weyerhaeuser Company
Federal Way, WA 98063-9777 (US)**

(72) Inventor: **Hansen, Michael R.
Seattle Washington 98155 (US)**

(74) Representative: **Trueman, Lucy Petra
Barker Brettell,
138 Hagley Road,
Edgbaston
Birmingham B16 9PW (GB)**

(54) **Polysaccharide treated cellulose fibers**

(57)    Wet laid webs of cellulose fibers are treated with a fiber treatment composition that includes a polysaccharide. The polysaccharide is introduced into the web using a fiber treatment composition and a process that produces a wet laid web that exhibits desirable tackiness properties between adjacent sheets and within the web. Airlaid webs produced from the wet laid webs of the present invention are useful in absorbent articles such as diapers, feminine hygiene products, and wipes.

*Fig. 1*

**Description**

FIELD OF THE INVENTION

The present invention relates to cellulose fibers that have been treated with a fiber treatment composition containing a polysaccharide in order to modify the properties of the cellulose fibers and to methods for applying polysaccharide containing fiber treatment compositions to cellulose fibers.

BACKGROUND OF THE INVENTION

Cellulose fibers have found widespread application in absorbent articles, such as diapers and feminine hygiene products. The cellulose fibers are generally used as an absorbent medium to acquire, transport, and hold fluids. While cellulose fibers are effective at acquiring, transporting, and holding fluids, many improvements to cellulose fibers have been made over the past decades to improve the performance properties of cellulose fibers in absorbent products. For example, cellulose fibers have been treated with various polymeric or nonpolymeric materials to impart to the fiber the ability to bind superabsorbent particles which are typically combined with cellulose fibers in the manufacture of diapers. For example, U.S. Patent No. 5,789,326 describes various families of polymeric and nonpolymeric materials useful for application to cellulose fibers in order to impart superabsorbent particle binding properties to cellulose fibers.

In addition to enabling cellulose fibers to bind superabsorbent particles or other particles, in some.instances, the materials described in U.S. Patent No. 5,789,326 as being useful for imparting a particle binding property to cellulose fibers also modify the densification properties of the fibers. For example, U.S. Patent Nos. 6,340,411 and 5,547,541 describe that webs of cellulose fibers treated with certain polymeric and nonpolymeric materials require less heat and pressure to densify the web to a given density as compared, to the heat and pressure needed to densify a similar web without the polymeric or nonpolymeric material present.

The cellulose fibers treated with the compositions described in U.S: Patent No. 5,47,541 are manufactured by applying the desired compositions to a wet laid web of cellulose fibers which has been produced, for example, using a Fourdrinier machine. The treated wet laid web of cellulose fibers is generally formed into a roll for bulk delivery to an absorbent product manufacturer. The absorbent product manufacturer typically unrolls the roll and processes the web in a fiberization unit that individualizes the fibers and prepares them for further processing.

Several challenges are faced by fiber manufacturers when producing the treated cellulose fibers discussed above. Depending on the use to which the fibers are put, the customer desires that the treatment chemistry be applied uniformly to the fibers. Thus, the particular chemistry which is applied to the cellulose fibers in the wet laid sheet manufacturing line should be capable of being readily dispersed within or impregnated into the wet laid web of cellulose fibers preferably to a level such that a sufficient number of fibers are treated to impart the desired properties to the product made from them. In addition, when the treated sheets are formed into a roll, it is undesirable to have the overlapping layers of the wet laid sheet stick together. When such sticking occurs, customers often consider the roll unusable. Likewise, it is undesirable for the chemistry applied to the wet laid web to increase the adhesion between individual fibers in a given layer of the roll. An increase in adhesion between individual fibers increases the energy needed to fiberize the wet laid web.

The absorbent product industry is a competitive industry where there is constant downward pressure on the cost of raw materials. The search for desirable treatment chemicals is limited by the need of the industry to use chemicals which are safe and which are not susceptible to a negative perception by the consuming public.

U.S. Patent No. 3,903,889 describes a process for adhering absorbent particles to pulp fibers using syrup, honey, and other polysaccharides such as dextrins. A specific example of a suitable polysaccharide is described as being corn syrup. U.S. Patent No. 5,789,326 which discusses U.S. Patent No. 3,903,889 notes that corn syrup is not a hygroscopic material and that com syrup is excluded as an acceptable binder for attaching superabsorbent particles to fibers in some embodiments because corn syrup remains tacky upon drying. U.S..Patent No. 5,789,326 also describes that such tacky binders make processing of binder coated fibers difficult. For example, neat corn syrup does not lend itself to ready introduction into a wet laid web of fibers. In addition, the application of neat com syrup to fibers causes the fibers to readily stick together. Heretofore, corn syrup has not found widespread use in the manufacture of customized cellulose fibers which have been treated to modify their properties, despite the favorable economics of using corn syrup.

SUMMARY OF THE INVENTION

The present invention solves problems encountered in introducing polysaccharides such as corn syrup into a wet laid web of cellulose fibers. The invention provides cellulose fiber based products that include a fiber treatment composition comprising polysaccharides and provides processes for producing such products that successfully overcome the problems introduced by the tackiness of many polysaccharides. The products and processes of the present invention provide a less costly alternative to treated cellulose fibers that employ more expensive treatment chemicals. At the same time, the present invention provides cellulose fibers that exhibit particle binding properties and densification properties that are similar to products that are produced using more expensive materials.

In one aspect, the present invention relates to a wet laid web of cellulose fibers that is useful in the preparation of an airlaid mass of fibers. The wet laid web of fibers includes cellulose fibers, and a fiber treatment composition that includes a polysaccharide and at least one agent having hydrogen bonding functionality. The fiber treatment composition is distributed within the wet laid web of cellulose fibers. In particular embodiments, the cellulose fibers are wood pulp fibers, the polysaccharide is corn syrup, the agents having hydrogen bonding functionality in the fiber treatment composition are sorbitol and propylene glycol, and the weight ratio of corn syrup to the combined weight of sorbitol and propylene glycol in the wet laid web is less than about 1:1.

In another particular embodiment, the polysaccharide is corn syrup, and the agents having hydrogen bonding functionality in the fiber treatment composition are lactic acid and propylene glycol. In particular aspects of this embodiment of the present invention, the cellulose fibers are wood pulp fibers and the weight ratio of corn syrup to the combined weight of the lactic acid and propylene glycol in the wet laid web is less than about 1:1.

In another embodiment of this aspect of the present invention, the wet laid web of cellulose fibers comprises cellulose fibers, a fiber treatment composition including a polysaccharide and at least one agent having hydrogen bonding functionality. The fiber treatment composition is distributed within the wet laid web of cellulose fibers and the polysaccharide is present in the web in an amount less than about 10 wt. % based on the dry weight of the treated web.

The foregoing wet laid web of cellulose fibers are useful in numerous applications, for example, in the preparation of an airlaid mass of fibers that is used in absorbent products such as diapers, feminine hygiene products, wipes, and the like.

The treated cellulose fibers of the wet laid web can be useful for binding particles to the fibers, such as superabsorbent particles. In addition, the treated cellulose fibers are useful in products where it is desired to reduce the energy needed to densify a mass of cellulose fibers to a particular density and to have cellulose fibers maintain a particular density once that particular density is achieved. In these applications, the wet laid webs of cellulose fibers are fiberized prior to being formed into the end product.

In another aspect, the present invention provides an absorbent product that includes cellulose fibers and the fiber treatment compositions described above. In this aspect, the polysaccharide is present on the fibers in an amount less than about 10 wt. % based on the dry weight of the treated cellulose fibers. In another embodiment of this aspect of the present invention, the absorbent fibers product includes cellulose fibers and a fiber treatment composition that includes a polysaccharide and at least one agent having hydrogen bonding functionality. In this embodiment, the polysaccharide is present on the fibers in an amount less than about 10 wt. % based on the dry weight of the treated cellulose fibers.

The present invention also provides a method for producing cellulose fibers that have been treated with a polysaccharide that includes the steps of providing a wet laid web of cellulose fibers and applying a fiber treatment composition that includes a polysaccharide and at least one agent having hydrogen bonding functionality. In particular embodiments, the polysaccharide is com syrup, and the agents having hydrogen bonding functionality in the fiber treatment composition are lactic acid and propylene glycol together; or sorbitol and propylene glycol together.

Wet laid webs of the present invention advantageously do not stick to each other when formed into a roll or bale. Adhesion between adjacent layers is considered undesirable because it complicates processing of the rolls or bales.

In another embodiment, the present invention relates to a roll or bale formed from a wet laid web of cellulose fibers for use in the preparation of an airlaid mass of fibers where the roll comprises cellulose fibers and a fiber treatment composition. The fiber treatment composition includes a polysaccharide and at least one agent having hydrogen bonding functionality. The fiber treatment composition being distributed within the wet laid web of cellulose fibers with adjacent layers in the roll or bale being substantially free of adhesion therebetween.

As noted above, the wet laid webs of treated cellulose fibers of the present invention can be further processed to individualize the fibers for use in the production of absorbent products such as diapers, feminine hygiene products, wipes, and the like, some of which include superabsorbent particles. The resulting fibers are useful in methods for binding superabsorbent particles to fibers and in methods for improving the densification properties of cellulose fibers. Accordingly, the present invention is also directed to fibrous products made from the fibers produced by any of the methods described herein and to absorbent products comprised of the cellulose fibers derived from the treated wet laid webs of the present invention.

BRIEF DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:

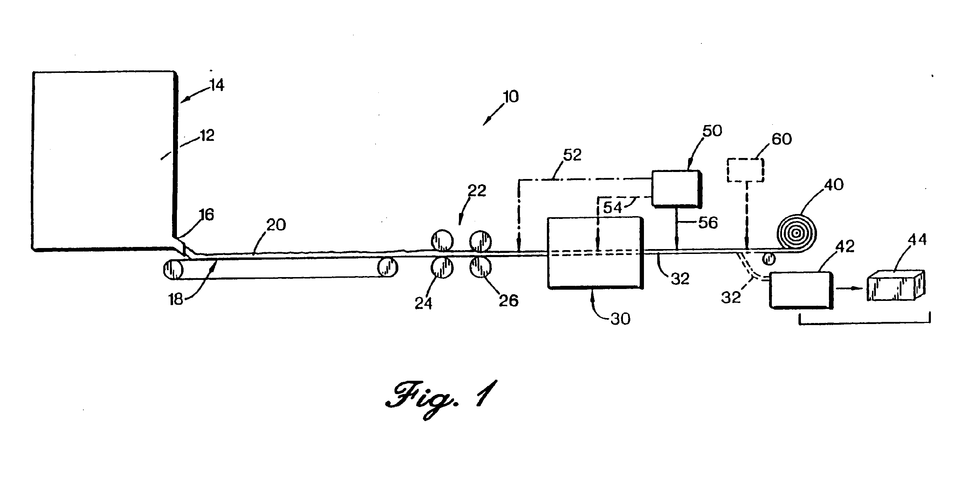

FIGURE 1 is a schematic illustration of a wet laid web manufacturing line illustrating the application of a fiber treatment composition to wet laid web in accordance with the present invention.

DETAILED DESCRIPTION OF THE PREFERRED EMBODIMENT

Wet laid webs of cellulose fibers formed in accordance with the present invention include cellulose fibers and a fiber treatment composition comprising a polysaccharide and at least one agent, and in specific embodiments at least two agents, having hydrogen bonding functionality. Exemplary cellulose fibers include those obtained from plant sources such as cotton, flax, bagasse, hemp, jute, rice, wheat, bamboo, corn, sisal, kenaf, peat moss, and the like. Preferred cellulose fibers are wood pulp fibers such as those described in U.S. Patent No. 5,789,326. Generally, such wood pulp fibers can be produced by a chemical, thermomechanical, or chemithermomechanical process. Suitable wood pulp fibers may also be pretreated prior to the application of the fiber treatment composition in accordance with the present invention. Examples of suitable pretreatments include crosslinking the fibers, treating the fibers to effect their wetability, or bleaching the fibers. Additionally, other fibers, natural or synthetic, may be included in the wet laid web. Examples of other fibers include silk, wool, linen, rayon, lyocell, polyethylene, polypropylene, polyester, and polyamide.

Polysaccharides comprise a combination of repeating monosaccharides linked together by glycosidic bonds. Polysaccharides useful in the present invention include water soluble polysaccharides such as corn syrup, honey, dextrin, and the like. Other useful polysaccharides may include molasses, starches, pectins, amyloses, and the like. The particular polysaccharide chosen for use in the present invention can be selected taking into consideration the cost of the polysaccharide as well as its impact on the ability of the other components of the fiber treatment composition to impart particle binding properties and/or densification properties to the cellulose fibers to which the fiber treatment composition is applied. Corn syrup is an economically favorable polysaccharide available from numerous commercial sources, typically as a solution containing about 80% solids. The selection of a particular corn syrup should take into consideration the tackiness of the corn syrup and its tendency to brown when exposed to heat. The corn syrup should not be so tacky that it is difficult to mix the corn syrup with the other components of the fiber treatment composition or causes the fiber treatment composition to be too viscous so as to impede the application of the fiber treatment composition to the wet laid web of cellulose fibers and the distribution of the fiber treatment composition into the web of cellulose fibers. If the corn syrup is too viscous to effectively mix with the other components of the fiber treatment composition, water can be added to reduce its viscosity. A suitable corn syrup for use in the present invention has a dextrose equivalent ranging from about 20 to about 50.

In accordance with one aspect of the present invention, the polysaccharide is combined with at least one agent having hydrogen bonding functionality, and in particular embodiments with at least two such agents, to provide the fiber treatment compositions used in the present invention. These agents can be either polymeric or nonpolymeric chemicals that have at least one functional group that is capable of forming hydrogen bonds. A plurality of suitable agents having hydrogen bonding functionality are described in U.S. Patent No. 5,641,561; U.S. Patent No. 5,789,326; and U.S. Patent No. 5,547,541 with reference to various polymeric binders and nonpolymeric binders. These discussions regarding the polymeric binders and nonpolymeric binders and their ability to bind particles and effect the densification properties of cellulose are expressly incorporated herein by reference. As described therein, the polymeric and nonpolymeric binders impart particle binding properties to cellulose fibers, and in some situations, modify the densification properties of fibers treated with such binders. Particular families of agents having hydrogen bonding functionality useful in the present invention include alcohols, hydroxy acids, and polycarboxylic acids. One reason these families of agents having hydrogen bonding functionality are desirable is due to their general acceptance by customers of the products of the present invention. Each of the foregoing family of materials includes functionality that allows the material to form hydrogen bonds, for example, with the cellulose fibers that the fiber treatment composition is applied to. In addition, it is advantageous, though not required, that a particular agent having hydrogen bonding functionality reduce the viscosity of the polysaccharide. Without intending to be bound by theory, it is believed that the particular embodiments of the agents having hydrogen bonding functionality useful in the present invention are capable of interacting with the hydrogen bonding functionality of the cellulose fibers so as to reduce the hydrogen bonding that occurs between the polysaccharide and the cellulose fibers, thus facilitating the distribution of the polysaccharide into the wet laid web of cellulose fibers. In instances where the agents having hydrogen bonding functionality do not provide a desired reduction in the viscosity of the polysaccharide, water can be added to the fiber treatment composition or its components in order to reduce the viscosity of the fiber treatment composition. When adding water to the fiber treatment composition, consideration must be given to not introducing an excessive amount of water into the wet laid fiber sheet so as to increase the risk of degradation of the treated wet laid web of cellulose fibers.

Specific examples of alcohols and hydroxy acids that are useful in the present invention include sorbitol, propylene glycol, lactic acid and its salt sodium lactate. The following discussion proceeds with reference to these specific examples of agents having hydrogen bonding functionality, however, it should be understood that the present invention is not so limited. The following description also discusses the present invention with reference to the specific polysaccharide corn syrup; however, it should be understood that the present invention is not so limited. In addition, while specific embodiments of the present invention are described in the context of a fiber treatment composition that includes at least two agents having hydrogen bonding functionality, the present invention is not necessarily limited to fiber treatment compositions that require at least two agents having hydrogen bonding functionality. Given the disclosure of the types of agents having hydrogen bonding functionality useful in the present invention, fiber treatment compositions employing at least one agent having hydrogen bonding functionality are within the scope of the present invention. A fiber treatment composition that includes a polysaccharide and at least one agent having hydrogen bonding functionality in accordance with the present invention can be applied to a wet laid web of cellulose fibers to produce a treated wet laid web which when rolled or formed into a bale is substantially free of adhesion or resistance to separation between adjacent layers.

A fiber treatment composition of the present invention is formed by mixing combinations of the foregoing components as described below in more detail. Water may be added to the mixture depending on the water content of the various components. In accordance with the present invention, it is desirable to maintain the overall water content of the fiber treatment composition below about 35 wt. % in order to avoid applying too much water to the wet laid web of fibers. Applying an excessive amount of water to the wet laid web of fibers increases the risk that degradation of the fibers will occur through decomposition or mold formation, particularly when the web of fibers is formed into a roll.

The amount of the fiber treatment composition applied to the wet laid web of fibers can vary depending upon the particular end application desired for the fibers. An amount of less than about 9 wt. % fiber treatment chemistry based on the dry weight of the treated wet laid web is exemplary. A more specific range of amounts is about 3 wt. % to about 9 wt. %, and an even more specific range is about 5 wt. % to about 9 wt. %. Larger amounts of fiber treatment chemistry can be applied to the web; however, the amount of fiber treatment chemistry applied must be balanced against the cost of applying such chemicals as well as the impact that increasing the level of the fiber treatment chemistry has on the adhesion between layers of the wet laid web when it is rolled and the adhesion that occurs between the fibers within the web. The amount of the fiber treatment composition applied to the wet laid web of fibers should not be so great that when the wet laid web is formed into a roll, adhesion occurs between adjacent layers in the roll. In addition, the amount of fiber treatment composition applied to the wet laid web of fibers should not be so great that it increases the adhesion between fibers, bonding them within the web, such that the amount of energy needed to defiberize the web becomes excessively high. Rolls or bales of the treated wet laid webs of the present invention are unique in that they are substantially free of adhesion between adjacent layers in the roll or bale. Preferably, the adjacent layers would simply "fall apart" upon unrolling or unstacking. Evaluation of the adhesion between adjacent layers in a roll or bale of a treated wet laid web of cellulose fibers can be evaluated using the stick test described below and the adhesion between fibers within a layer of the wet laid web of cellulose fibers can be evaluated using the fiberization energy test needed to break up the web as described below in more detail.

In accordance with the present invention, the amount of the fiber treatment composition applied to the wet laid web and the composition of the fiber treatment composition can be selected so that the amount of corn syrup applied to the wet laid web ranges from about 0.1 to about 4.5 wt. % based on the dry weight of the treated fibers. Amounts of corn syrup less than 4.5 wt. %, such as about 1.0 to about 4.0 wt. % have produced satisfactory results. Amounts of corn syrup greater than 4.5 wt. % up to less than 10%, for example up to about 7 wt. % may be suitable provided they do not cause the treated wet laid web to fail the stick test or fiberization energy test.

The amount of propylene glycol applied to the wet laid web of fibers can vary. An exemplary range of the amount of propylene glycol that can be applied to the wet laid web of cellulose fibers ranges from about 0.1 to about 4.5 wt. % based on the dry weight of the treated fibers. Amounts of propylene glycol less than 4.5 wt. %, such as about 1.0 wt. % to about 4.0 wt. % have produced satisfactory results. Amounts of propylene glycol greater than 4.5 wt. % may be suitable. However, amounts greater than about 15 wt. % increase costs while providing no additional enhancement of fiber properties.

The amount of sorbitol applied to the wet laid web of fibers may vary as well. An exemplary range of the amount of sorbitol that can be applied to the wet laid web of fibers is 0 to about 4.5 wt. % based on the dry weight of the treated fibers. Amounts of sorbitol less than 4.5 wt. %, such as about 1.0 wt. % to about 3.5 wt. % have produced satisfactory results. The amount of sorbitol applied to the wet laid web of fibers may be greater than 4.5 wt. %; however, increasing amounts of sorbitol increases costs.

Lactic acid may also be present in the fiber treatment composition useful in the present invention and may be present in its acid form or its sodium salt form (sodium lactate) in an amount ranging from about zero to about 5.0 wt. % based on the dry weight of the treated fibers. When lactic acid and sodium lactate are used, the ratio of the acid to salt may vary, with an exemplary ratio being 3:1.

When the fiber treatment composition is applied to the wet laid web in an amount of about 9 wt. % based on the dry weight of the treated wet laid web, the amount of corn syrup in the fiber treatment composition can range from about 1.0 wt. % to about 50 wt. % based on the weight of the non-aqueous components in the fiber treatment composition. The amount of propylene glycol present in the fiber treatment composition can range from about 1.0 wt. % to about 50 wt. % and the amount of sorbitol present in the fiber treatment composition can range from about 0 wt. % to about 50 wt. %. When lactic acid and sodium lactate are employed, lactic acid may be present in an amount ranging from about 0 wt. % to about 55 wt. % and sodium lactate can be present in an amount ranging from about 0 wt. % to about 30 wt. % in the fiber treatment composition. In particular embodiments, the weight ratio of the corn syrup to the combined weight of sorbitol and propylene glycol in the fiber treatment composition is less than about 1:1 down to about 1:5 or less. In another particular embodiment, the weight ratio of corn syrup to the combined weight of lactic acid and propylene glycol in the fiber treatment composition is less than about 1:1 to as low as 1:5 or less. As with the wt. % mentioned earlier in this paragraph, the foregoing weight ratios are based on the weight of the non-aqueous components in the fiber treatment composition. It should be understood that as the amount of the fiber treatment composition applied varies, the amount of the various components in the fiber treatment composition can change in order to achieve the desired levels of agent loading described above.

In accordance with the present invention, the fiber treatment compositions described above are applied to a wet laid sheet of cellulose fibers that has been produced as explained in more detail in the following paragraphs. The particular method of application is not critical provided that the application method results in a distribution of the fiber treatment composition within the wet laid sheet of cellulose fibers. Distribution of the fiber treatment composition within the wet laid sheet of cellulose fibers is not intended to require that the fiber treatment composition be uniformly distributed throughout the wet laid sheet of cellulose fibers, although this is preferred. Furthermore, distribution of the fiber treatment composition within the wet laid sheet of cellulose fibers does not require that the fiber treatment composition be distributed throughout the entire volume of the wet laid sheet of cellulose fibers. Exemplary techniques for applying the fiber treatment chemistry include streaming, spraying, curtain coating, or rolling the fiber treatment composition onto one, or both, surfaces of the wet laid web of cellulose fibers, or immersing the wet laid web in a bath of the fiber treatment composition.

FIGURE 1 illustrates a wet laid sheet manufacturing line such as a pulp sheet manufacturing line 10. In this manufacturing line, a pulp slurry 12 is delivered from a headbox 14 through a slice 16 and onto a Fourdrinier wire 18. The pulp slurry 12 typically includes wood pulp fibers and may also include synthetic or other non-cellulose fibers as part of the slurry. Water is drawn from the pulp deposited on wire 18 by a conventional vacuum system, not shown, leaving a deposited pulp sheet 20 which is carried through a dewatering station 22, illustrated in this case as two sets of calendar rolls 24, 26 each defining a respective nip through which the pulp sheet or mat 20 passes. From the dewatering station, the pulp sheet 20 enters a drying section 30. In a conventional pulp sheet manufacturing line, drying section 30 may include multiple canister dryers with the pulp mat 20 following a serpentine path around the respective canister dryers and emerging as a dried sheet or mat 32 from the outlet of the drying section 30. Other alternate drying mechanisms, alone or in addition to canister dryers, may be included in the drying stage 30. The dried pulp sheet 32 has a maximum moisture content pursuant to the manufacturer's specifications. Typically, the maximum moisture content is no more than 10% by weight of the fibers and most preferably no more than about 6% to 8% by weight. Unless overly damp fibers are immediately used these fibers are subject to degradation by, for example, mold or the like. The dried sheet 32 is taken up on a roll 40 for transportation to a remote location, that is, one separate from the pulp sheet manufacturing' line, such as at a user's plant for use in manufacturing products. The dried pulp sheets have a basis weight of about 200 g/m$^2$ to about 1000 g/m$^2$ or more and a density on the order of at least about 0.5 g/cm$^3$ to about 1.2 g/cm$^3$. Dried pulp sheets having the foregoing basis weights are structurally distinct form lighter basis weight sheets of wet laid or airlaid wood pulp fibers such as tissue paper, paper towels, or other types of paper-like wet laid or airlaid webs of cellulose fibers. Alternatively, the dried sheet 32 is collected in a baling apparatus 42 from which bales of the pulp 44 are obtained for transport to a remote location.

A fiber treatment composition of the type explained in detail above is applied to the pulp sheet from one or more fiber treatment composition applying devices, one of which is indicated at 50 in FIGURE 1. Any applying device may be used, such as streamers, sprayers, roll coaters, curtain coaters, immersion applicators, or the like. Sprayers are typically easier to utilize and incorporate into a pulp-sheet manufacturing line. As indicated by the arrows 52, 54, and 56, the fiber treatment composition may be applied at various locations or at multiple locations on the pulp sheet manufacturing line, such as ahead of the drying stage 30 (indicated by line 52), intermediate the drying stage 30 (as indicated by line 54), or downstream from the drying stage 30 (as indicated by the line 56). At location 52, the water remaining in the sheet or mat 20 at this stage tends to interfere with the penetration of the binder into the sheet. Consequently, application of the fiber treatment composition after some drying has taken place, for example at location 54, is preferable. If the fiber treatment composition is applied at location 56 in an amount which would cause the moisture content of the sheet to exceed the desired maximum level, an additional drying stage (not shown) may be included in the pulp manufacturing line to bring the moisture content down to the desired level.

The rolls 40 or bales 44 of the treated wet laid web of fibers may be transported to a remote location for use by a user. These rolls or bales are then refiberized by a fiberizing device, such as a hammermill which may be used alone or in conjunction with other devices such as picker rolls or the like for breaking up the sheet 32 or bales 42 into individual fibers. Depending on the end use, the individualized fibers may be combined with particulate material, such as superabsorbent particles, and/or airlaid into a web and densified.

With this approach, the end user of the treated fibers may readily select particles to be combined with the fibers. The user has flexibility in air laying or otherwise processing the treated fibers of the present invention into a finished product.

As discussed above, the sheets of wet laid web of cellulose fibers treated with the fiber treatment compositions that include polysaccharides in accordance with the present invention do not stick to each when the sheet is formed into a roll. Sticking together of the sheets is undesirable because it complicates subsequent processing of the roll. In extreme situations, the roll can in essence become an unusable "brick" of fibers.

One method of assessing the relative tendency of wet laid webs treated with different fiber treatment compositions to stick together is described below.

STICK TEST

At least two 5 x 12 inch sections of a wet laid web of cellulose fibers, 5 inches x 12 inches, are impregnated with the fiber treatment composition being evaluated. The wet laid web of cellulose fibers was a product designated NB 416 from the Weyerhaeuser Company. The sections of treated web are then placed on a flat surface, one on top of the other, with the treated side of one sheet facing the untreated side of the other sheet, in much the same fashion as they would be juxtaposed in a roll of the product. A flat aluminum plate covering the entire surface of the top sheet is placed on the top sheet and a 20 kg weight is placed in the center of the aluminum plate, so as to distribute the mass evenly over the entire surface of the sheets. The weight is left in place for approximately 12 hours, after which it is removed and an attempt is made to separate the sections of the treated pulp sheet. Ready separation of the treated pulp sheets with no resistance is deemed satisfactory, any resistance to separation is deemed unsatisfactory. Resistance to separation was evaluated visually by observing the surfaces of adjacent layers as they were separated. The presence of picking or an insubstantial number of fibers being pulled from the surface of one of the layers was considered an indication of resistance to separation.

As discussed above, the present invention also introduces a polysaccharide such as corn syrup into a wet laid web of cellulose fibers, without having the fibers in a given layer of the wet laid web stick to each other excessively. The tendency of fibers in a layer of the wet laid web to stick to each other is undesirable because it increases the fiberization energy needed to defiberize the wet laid web into individualized fibers for further processing as discussed above. Increases in fiberization energy increase energy costs for the end user. In addition, the excessive fiber to fiber adhesion can negatively impact the quality of the fibers after they have been defiberized. One method of assessing the fiberization energy required to defiberize wet laid webs treated with different fiber treatment compositions is described below.

III. FIBERIZATION ENERGY

A lab scale Kamas hammermill was employed to assess the fiberization energy required to fiberize a pulp sheet treated with various fiber treatment compositions. The pulp sheet sample was cut into 5 cm-wide strips about 46 cm long. A sufficient number of strips were used to provide a total of about 100 to 150 g of fiberized pulp sheet. The strips of pulp sheet are fed at a rate of about 2.8 g/sec. to the hammermill feed roller. The motor speed is set at about 3,000 rpm. For 46 cm strips, a sample one time of about 5.5 sec. is appropriate. The pulp sheets are fed to the hammermill for the set time period. The energy digital readout displays the energy required as measured in watt hours needed to fiberize the pulp sheet. The rate of fibers fiberized is determined by subtracting the weight of the remaining pulp sheets not processed from the initial weight of the pulp sheets fed to the hammermill. The fiberization energy is determined using the following formula:

$$\frac{3{,}600 \; X \; Energy \; (wH)}{Fiberzed \; Weight \; (g)} = Energy \; (kJ/kg)$$

The wet laid web of cellulose fibers carrying the fiber treatment composition as described above is useful in the preparation of an airlaid mass of fibers that can be used in an absorbent products such as a diaper, feminine hygiene product, wipes, bandages, or the like. Certain embodiments of the present invention produce fibers capable of binding particles, such as superabsorbent particles. In other embodiments, fibers are provided that do not bind particles as greatly as other embodiments yet exhibit desirable properties with respect to ease of densification, energy required to densify the fibers and the ability of the fibers to remain densified once pressed. The particular end use to which the fibers of the present invention will be put will depend upon the desires of the customer purchasing the wet laid web of cellulose fibers formed in accordance with the present invention.

The following examples illustrate particular embodiments of the present invention and the ability of the fibers of the present invention to bind particles and to be densified. Also, the examples illustrate how the wet laid webs of cellulose fibers formed in accordance with the present invention perform under the stick test and fiberization energy test described above.

The following fiber treatment compositions, some of which are controls and others which are illustrative of the present invention, were used in the following examples.

TABLE 1

| FIBER TREATMENT COMPOSITION (WT. % BASED ON NON-AQUEOUS COMPONENTS) | | | | | | |
|---|---|---|---|---|---|---|
| SAMPLE DESIGNATION | CORN SYRUP | SORBITOL | PROPYLENE GLYCOL | LACTIC ACID | SODIUM LACTATE | % WATER |
| 36 A (Comparative) | 100 | --- | --- | --- | --- | 25 |
| 36 B (Comparative) | 100 | --- | --- | --- | --- | 35 |
| Control A | --- | 40 | 17 | 32 | 11 | 30 |
| Control B | --- | 70 | 30 | --- | --- | 30 |
| 36 C | 50 | 25 | 25 | --- | --- | 25 |
| 36 D | 50 | 25 | 25 | --- | --- | 30 |
| 36 E | 33 | 33 | 33 | --- | --- | 30 |
| 36 F | 33 | 33 | 33 | | | 30 |
| 36 G | 40 | --- | 17 | 32 | 11 | 30 |
| 42 H | 20 | 20 | 17 | 32 | 11 | 30 |

The corn syrup employed in the fiber treatment compositions above which are designated with the "36" prefix had a dextrose equivalent of 36. For samples designated with the "42" prefix, the corn syrup had a dextrose equivalent of 42. Both corn syrups had a water content of about 20%. Water was added to reduce the solids content to about 70%, making the corn syrup easier to mix with the other components. The sorbitol employed had a water content of about 30%, the propylene glycol had a water content of about 0.5%, the lactic acid had a water content of about 12-15%, and the sodium lactate had a water content of about 40%. When necessary, water was also added to the fiber treatment composition to provide the indicated percentage of water content.

EXAMPLE 1

STICK TEST RESULTS

The stick test described above was carried out on samples of a wet laid web of cellulose fibers (NB 416) having a basis weight of approximately 750 $g/m^2$, and a density of about 750 $g/m^2$, that had been treated with certain comparative fiber treatment compositions, or fiber treatment compositions of the present invention. The comparative samples are not examples of the present invention. The chemical added to the pulp web was 9 wt. % based on the dry weight of the treated wet laid web. The results of the stick test are set forth below.

TABLE 2

| STICK TEST RESULTS | |
|---|---|
| SAMPLE | PASSED |
| 36 A (Comparative) | No |
| 36 B (Comparative) | No |

TABLE 2   (continued)

| STICK TEST RESULTS | |
|---|---|
| SAMPLE | PASSED |
| 36 C | Yes |
| 36 D | Yes |
| 36 E | Yes |
| 36 F | Yes |
| 36 G | Yes |
| 42 H | Yes |

The results of the stick test show that the comparative samples that employ a fiber treatment composition that includes only corn syrup, do not pass the stick test. The samples of the present invention which include com syrup in combination with at least one agent having hydrogen bonding functionality passed the stick test.

EXAMPLE 2

**FIBERIZATION ENERGY TEST RESULTS**

The fiberization energy test was carried out on samples of a wet laid web (NB 416) having a basis weight of about 750 g/m$^2$, and a density of 750 g/m$^2$, that had been treated with a fiber treatment composition add-on of 9 wt. % based on the dry weight of the treated wet laid web. The results of the fiberization energy test are set forth in Table 3.

TABLE 3

| FIBERIZATION ENERGY TEST RESULTS | |
|---|---|
| SAMPLE | ENERGY REQUIRED (kJ/kg) |
| 36 A (Comparative) | 126 |
| 36 B (Comparative) | 137 |
| 36 C | 143 |
| 36 D | 117 |
| 36 E | 113 |
| 36 F | 112 |
| 36 G | 115 |
| 42 H | 109 |

The results of the fiberization energy tests indicate the amount of energy needed to break down the fiber to fiber adhesion and fiberize the respective samples. The results indicate that wet laid webs of cellulose fibers treated with a fiber treatment composition comprising a polysaccharide and at least one agent having hydrogen bonding functionality in accordance with the present invention exhibit fiberization energy properties that are similar to the fiberization energy needed to fiberize the comparative samples.

PARTICLE RETENTION

Individualized fibers prepared from two commercially available wet laid webs of cellulose fibers and individualized fibers prepared from a wet laid web of cellulose fibers treated with a fiber treatment composition comprising a polysaccharide and at least one agent having hydrogen bonding functionality were tested to evaluate their ability to retain superabsorbent particles. Samples of untreated webs, webs of pulp treated with nonpolysaccharide containing formulations, and formulations of the present invention were individually fed into a Fitz hammermill, fiberized, mixed with superabsorbent (SXM 77 available from Stockhausen, of Greensboro, North Carolina) and airlaid on a piplot scale (ca. 12 inch wide) M & J airlay machine (available from M & J, of Horsens, Denmark). The superabsorbent was added at a rate to yield a mixture of fibers and particles that was 40% superabsorbent, based on the weight of the fibers and particles. The pulp feed and M & J airlay line were run at speeds to yield a fluff/superabsorbent web that was approximately 450 g/m$^2$. Ten centimeter by ten centimeter sections of each web were punched out, weighed and placed in a column of decreasing hole size sieves (Numbers 4, 20, 200, and pan). The column of sieves and sample were then subjected to lateral shaking and simultaneous z-direction tapping motions for five minutes. Various fractions, caught in each of the sieves, were then weighed. The fractions in the lower two sieves were, in some cases, composed of two parts -- loose fibers and unattached superabsorbent particles. When present, those two fractions were weighed separately and the relative amount of unattached particles was assessed by the following equation:

%loose SAP = grams loose SAP/(initial sample mass X 0.4)

The results are presented below in Table 4.

TABLE 4

| SUPERABSORBENT PARTICLE (SAP) RETENTION | |
| --- | --- |
| SAMPLE | % Loose SAP |
| NB 416 (Control) | 75.88 |
| Control A | 1.85 |
| 36 G | 1.81 |

This example illustrates how individualized fibers prepared from a wet laid web of cellulose fibers of the present invention are capable of retaining superabsorbent particles.

DENSIFICATION

The densification properties of individualized fibers prepared from commercially available wet laid webs of cellulose fibers and individualized fibers prepared from a wet laid web of the present invention were evaluated as described below.

Sheets of a commercially available wet laid web of wood pulp fibers designated as NB 416 from the Weyerhaeuser Company, Control B, and webs of NB 416 treated with fiber treatment compositions 36 E and 36 F were individualized in a hammermill. The individualized fibers were formed into 6-inch diameter pads using a pad-former. The pads were weighed to record an initial weight and then compressed between two chrome plates in a Carver press applying test pressures of 50 psi, 100 psi, and 150 psi. Once pressed, the test pressure was removed and the pad allowed to relax for approximately 20 seconds or until stabilized. The caliper of the densified pad was then taken and the density determined by the following formula:

$$Density\ (g\ /\ cm^3) = \frac{Weight\ of\ Pad\ (g)}{100cm^2\ (area\ of\ pad\ x\ caliper\ in\ cm)}$$

The results of this testing are set forth in Table 5 below.

TABLE 5

| DENSIFICATION PROPERTIES DENSITY (g/cm$^3$) | | | |
| --- | --- | --- | --- |
| SAMPLE | 50 psi | 100 psi | 150 psi |
| Control B | 0.159 | 0.250 | 0.360 |
| NB 416 (Control) | 0.117 | 0.177 | 0.231 |
| 36 E | 0.149 | 0.235 | 0.286 |
| 36 F | 0.142 | 0.221 | 0.271 |

The reported results indicate how individualized fibers prepared from wet laid webs of the present invention exhibit densification properties similar to those of the control sample B and improved densification properties relative to control sample NB 416.

While the preferred embodiment of the invention has been illustrated and described, it will be appreciated that various changes can be made therein without departing from the spirit and scope of the invention.

**Claims**

1. A wet laid web of cellulose fibers for use in the preparation of an airlaid mass of fibers, the wet laid web of fibers comprising:

   cellulose fibers;
   a fiber treatment composition including a polysaccharide and at least one agent having hydrogen bonding functionality, the fiber treatment composition being distributed within the wet laid web of cellulose fibers.

2. The wet laid web of cellulose fibers of Claim 1, wherein the fiber treatment composition comprises at least two agents having hydrogen bonding functionality.

3. The wet laid web of cellulose fibers of Claim 1, wherein the cellulose fibers are wood pulp fibers.

4. The wet laid web of cellulose fibers of Claim 1, wherein the fiber treatment composition includes no more than 35 weight percent water.

5. The wet laid web of cellulose fibers of Claim 1, wherein the web was a basis weight ranging from about 200 g/m$^2$ to about 1000 g/m$^2$.

6. The wet laid web of cellulose fibers of Claim 1, wherein the polysaccharide is selected from the group comprising corn syrup, honey, dextrins, molasses, starches, pectins, and amyloses.

7. The wet laid web of cellulose fibers of Claim 2, wherein the polysaccharide is corn syrup.

8. The wet laid web of cellulose fibers of Claim 7, wherein the agents having hydrogen bonding functionality are selected from the group comprising alcohols, hydroxy acids and polycarboxylic acids.

9. The wet laid web of cellulose fibers of Claim 8, wherein the agents having hydrogen bonding functionality comprise propylene glycol and sorbitol.

10. The wet laid web of cellulose fibers of Claim 9, wherein the weight ratio of corn syrup to the combined weight of sorbitol and propylene glycol in the wet laid web is less than about 1:1.

11. The wet laid web of cellulose fibers of Claim 10, wherein the weight ratio of corn syrup to the combined weight of sorbitol and propylene glycol in the wet laid web ranges from about 1:1 to about 1:5.

12. The wet laid web of cellulose fibers of Claim 7, wherein the com syrup is in the wet laid web of cellulose fibers in an amount ranging from about 0.01 to about 7 weight percent corn syrup solids based on the dry weight of the treated fibers.

13. The wet laid web of cellulose fibers of Claim 9, wherein the agents having hydrogen bonding functionality further comprise lactic acid.

14. The wet laid web of cellulose fibers of Claim 13, wherein the fiber treatment composition further comprises sodium lactate.

15. The wet laid web of cellulose fibers of Claim 8, wherein the agents having hydrogen bonding functionality comprise lactic acid and propylene glycol.

16. The wet laid web of cellulose fibers of Claim 15, wherein the fiber treatment composition further comprises sodium lactate.

**17.** An absorbent fibrous product comprising:

cellulose fibers; and
a fiber treatment composition on the cellulose fibers, the fiber treatment composition including a polysaccharide and at least one agent having hydrogen bonding functionality, the polysaccharide present on the fibers in an amount less than about 10 wt % based on the dry weight of the treated cellulose fibers.

**18.** The absorbent fibrous product of Claim 17, wherein the fiber treatment composition comprises at least two agents having hydrogen bonding functionality.

**19.** The absorbent fibrous product of Claim 17, further comprising superabsorbent particles.

**20.** An absorbent fibrous product of Claim 17, wherein the cellulose fibers are wood pulp fibers.

**21.** The absorbent fibrous product of Claim 18, wherein the polysaccharide is corn syrup.

**22.** The absorbent fibrous product of Claim 21, wherein the agents having hydrogen bonding functionality are selected from the group comprising alcohols, hydroxy acids and polycarboxylic acids.

**23.** The absorbent fibrous product of Claim 22, wherein the agents having hydrogen bonding functionality comprise propylene glycol and sorbitol.

**24.** The absorbent fibrous product of Claim 23, wherein the weight ratio of corn syrup to the combined weight of propylene glycol and sorbitol on the cellulose fibers ranges from 1:1 to about 1:5.

**25.** The absorbent fibrous product of Claim 24, wherein the corn syrup is on the cellulose fibers in an amount ranging from about 0.01 to about 7 weight percent corn syrup solids based on the dry weight of the treated fibers.

**26.** The absorbent fibrous product of Claim 23, wherein the agents having hydrogen bonding functionality further comprise lactic acid.

**27.** The absorbent fibrous product of Claim 26, wherein the fiber treatment composition further comprises sodium lactate.

**28.** The absorbent fibrous product of Claim 22, wherein the agents having hydrogen bonding functionality comprise lactic acid and propylene glycol.

**29.** The absorbent fibrous product of Claim 28, wherein the fiber treatment composition further comprises sodium lactate.

**30.** The absorbent fibrous product of Claim 17, wherein the product is a diaper or feminine hygiene product.

**31.** A method for producing a wet laid web of cellulose fibers comprising:

providing a wet laid web of cellulose fibers; and
applying a fiber treatment composition including a polysaccharide and at least one agent having hydrogen bonding functionality to the wet laid web of cellulose fibers.

**32.** The method of Claim 31, wherein the polysaccharide is corn syrup.

**33.** The method of Claim 32, wherein the fiber treatment composition comprises at least two agents having hydrogen bonding functionality.

**34.** The method of Claim 33, wherein the agents having hydrogen bonding functionality comprise propylene glycol and sorbitol.

**35.** The method of Claim 34, wherein the com syrup is applied to the wet laid web of cellulose fibers in a weight ratio of corn syrup to the combined weight of sorbitol and propylene glycol ranging from about 1:1 to about 1:5.

**36.** The method of Claim 34, wherein the corn syrup is applied to the wet laid web of cellulose fibers in an amount ranging from about 0.01 to about 7 weight percent corn syrup solids based on the dry weight of the treated fibers.

**37.** The method of Claim 34, wherein the agents having hydrogen bonding functionality further comprise lactic acid.

**38.** The method of Claim 37, wherein the fiber treatment composition further comprises sodium lactate.

**39.** The method of Claim 33, wherein the agents having hydrogen bonding functionality comprise lactic acid and propylene glycol.

**40.** The method of Claim 39, wherein the fiber treatment composition further comprises sodium lactate.

**41.** A roll or bale formed from a wet laid web of cellulose fibers for use in the preparation of an airlaid mass of fibers, the roll or bale comprising:

cellulose fibers; and
a fiber treatment composition including a polysaccharide and at least one agent having hydrogen bonding functionality, the fiber treatment composition being distributed within the wet laid web of cellulose fibers, adjacent layers of the wet laid web of cellulose fibers in the roll or bale being substantially free of adhesion therebetween.

**42.** The roll or bale of Claim 41 wherein the fiber treatment composition comprises at least two agents having hydrogen bonding functionality.

Fig. 1

## EUROPEAN SEARCH REPORT

**European Patent Office**

**Application Number**

EP 03 25 5804

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 770 711 A (GREENE SHARON LINDA ET AL) 23 June 1998 (1998-06-23)<br><br>* claims 1-21 *<br>--- | 1,3,6-8, 17, 20-22, 31,32 | D21C9/00 |
| X,D | US 3 903 889 A (TORR DECEASED DAVID) 9 September 1975 (1975-09-09)<br><br>* column 7, line 27-43; claims 1-6; figures 1-5 *<br>--- | 1,3,6-8, 17, 20-22, 31,32 | |
| X | US 4 693 713 A (DAHMEN KURT ET AL) 15 September 1987 (1987-09-15)<br><br>* claims 1-17; examples 3,5 *<br>--- | 1,3,6-8, 17, 20-22, 31,32 | |
| X | WO 01 48025 A (KIMBERLY CLARK CO) 5 July 2001 (2001-07-05)<br><br>* page 26, line 21 - page 32, line 18; claims 1-17 *<br>--- | 1,3,6-8, 17, 20-22, 31,32 | **TECHNICAL FIELDS SEARCHED (Int.Cl.7)**<br><br>D21C |
| A | EP 0 392 528 A (KIMBERLY CLARK CO) 17 October 1990 (1990-10-17) * the whole document *<br>--- | 1-42 | |
| A | WO 00 39389 A (WEYERHAEUSER CO) 6 July 2000 (2000-07-06) * the whole document *<br>--- | 1-42 | |
| A | WO 01 47569 A (KIMBERLY CLARK CO) 5 July 2001 (2001-07-05) * the whole document *<br>--- | 1-42 | |

-/--

The present search report has been drawn up for all claims

| Place of search | Date of completion of the search | Examiner |
|---|---|---|
| MUNICH | 28 November 2003 | Karlsson, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 03 25 5804

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | US 4 913 773 A (KNUDSEN KEITH W  ET AL) 3 April 1990 (1990-04-03) * the whole document * --- | 1-42 | |
| A | EP 0 440 472 A (JAMES RIVER CORP) 7 August 1991 (1991-08-07) * the whole document * ----- | 1-42 | |

TECHNICAL FIELDS SEARCHED      (Int.Cl.7)

The present search report has been drawn up for all claims

| Place of search | Date of completion of the search | Examiner |
|---|---|---|
| MUNICH | 28 November 2003 | Karlsson, L |

EPO FORM 1503 03.82 (P04C01)

## ANNEX TO THE EUROPEAN SEARCH REPORT
## ON EUROPEAN PATENT APPLICATION NO.

EP 03 25 5804

This annex lists the patent family members relating to the patent documents cited in the above-mentioned European search report.
The members are as contained in the European Patent Office EDP file on
The European Patent Office is in no way liable for these particulars which are merely given for the purpose of information.

28-11-2003

| Patent document cited in search report | | Publication date | Patent family member(s) | | Publication date |
|---|---|---|---|---|---|
| US 5770711 | A | 23-06-1998 | CA | 2213873 A1 | 30-03-1998 |
| US 3903889 | A | 09-09-1975 | NONE | | |
| US 4693713 | A | 15-09-1987 | DE | 3128100 A1 | 27-01-1983 |
| | | | AT | 16891 T | 15-12-1985 |
| | | | DE | 3267907 D1 | 23-01-1986 |
| | | | WO | 8300289 A1 | 03-02-1983 |
| | | | EP | 0071063 A1 | 09-02-1983 |
| | | | JP | 5081263 B | 12-11-1993 |
| | | | JP | 58501107 T | 14-07-1983 |
| | | | US | RE33839 E | 03-03-1992 |
| WO 0148025 | A | 05-07-2001 | AU | 2593501 A | 09-07-2001 |
| | | | BR | 0016787 A | 07-01-2003 |
| | | | DE | 10085362 T0 | 05-12-2002 |
| | | | GB | 2374345 A | 16-10-2002 |
| | | | WO | 0148025 A1 | 05-07-2001 |
| EP 0392528 | A | 17-10-1990 | US | 5161686 A | 10-11-1992 |
| | | | AU | 626655 B2 | 06-08-1992 |
| | | | AU | 5311890 A | 18-10-1990 |
| | | | CA | 2014204 A1 | 14-10-1990 |
| | | | EP | 0392528 A2 | 17-10-1990 |
| | | | JP | 3000057 A | 07-01-1991 |
| | | | ZA | 9002560 A | 27-03-1991 |
| WO 0039389 | A | 06-07-2000 | US | 6471824 B1 | 29-10-2002 |
| | | | AU | 2480400 A | 31-07-2000 |
| | | | WO | 0039389 A1 | 06-07-2000 |
| | | | US | 2003029585 A1 | 13-02-2003 |
| | | | US | 2003037890 A1 | 27-02-2003 |
| | | | US | 2003037891 A1 | 27-02-2003 |
| | | | US | 2003029586 A1 | 13-02-2003 |
| WO 0147569 | A | 05-07-2001 | AU | 2583701 A | 09-07-2001 |
| | | | BR | 0016828 A | 29-07-2003 |
| | | | CN | 1450918 T | 22-10-2003 |
| | | | EP | 1242128 A1 | 25-09-2002 |
| | | | JP | 2003524687 T | 19-08-2003 |
| | | | WO | 0147569 A1 | 05-07-2001 |
| | | | US | 2003139714 A1 | 24-07-2003 |
| US 4913773 | A | 03-04-1990 | NONE | | |
| EP 0440472 | A | 07-08-1991 | AT | 126556 T | 15-09-1995 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**                EP 03 25 5804

This annex lists the patent family members relating to the patent documents cited in the above-mentioned European search report.
The members are as contained in the European Patent Office EDP file on
The European Patent Office is in no way liable for these particulars which are merely given for the purpose of information.

28-11-2003

| Patent document cited in search report | Publication date | Patent family member(s) | Publication date |
|---|---|---|---|
| EP 0440472 A | | CA 2035402 A1 | 02-08-1991 |
| | | DE 69112089 D1 | 21-09-1995 |
| | | DE 69112089 T2 | 11-01-1996 |
| | | EP 0440472 A1 | 07-08-1991 |
| | | ES 2075339 T3 | 01-10-1995 |
| | | FI 910467 A | 02-08-1991 |

EPO FORM P0459